# EUROPEAN PATENT APPLICATION

(11) **EP 4 776 293 A1**
(43) Date of publication of application: **15.07.2026**
(21) Application number: 24862807.5
(22) Date of filing: 04.09.2024
(51) Int. Cl.: G16C 20/50

(54) **DESIGN ASSISTANCE DEVICE, DESIGN ASSISTANCE METHOD, PROGRAM, AND DESIGN ASSISTANCE SYSTEM**

(30) Priority: 08.09.2023 JP 2023145863
(71) Applicant: Resonac Corporation, Tokyo 105-7325 (JP)
(72) Inventor: KAKUDA, Kohsuke, Tokyo 105-7325 (JP); SAKAGUCHI, Suguru, Tokyo 105-7325 (JP)
(74) Representative: Strehl & Partner mbB
(86) International application number: PCT/JP2024/031652
(87) International publication number: WO 2025/053155

(57) **Abstract**

A design support device is provided for supporting design of a molecule that satisfies a desired physical property. The design support device includes an input reception unit configured to receive an input of information on a desired physical property; a search unit configured to search for information on a molecule that satisfies the desired physical property, using a database storing information on molecules; a candidate molecule generation unit configured to generate information on a plurality of candidate molecules from a base molecule when there is no information on the molecule that satisfies the desired physical property in the database; a prediction unit configured to predict information on the physical property for the information on the candidate molecules; and a presentation unit configured to select and present the candidate molecule that satisfies the desired physical property, based on the predicted information on the physical property.

## Description

### TECHNICAL FIELD

The present disclosure relates to a design support device, a design support method, a program, and a design support system.

### BACKGROUND

For example, a search for a molecule that satisfies desired physical properties is performed by searching a large number of molecules registered in a database or by predicting physical properties of a generated candidate molecule. Conventionally, a molecular design support system configured to take a molecular structural formula of a molecule to be designed as input and predict property values of the molecule of the input molecular structural formula is known (for example, see Patent Document 1).

### Related Art Document

### Patent Document

Patent Document 1: Japanese Laid-open Patent Application Publication No. 2021-117798

### SUMMARY OF THE INVENTION

### Problem to be solved by the invention

However, in a search for a molecule that satisfies desired physical properties in the related art, an operator performs a search using a tool for searching a database or the like or a tool for predicting physical properties of the molecule from a molecular structural formula, so that the amount of work to be performed by the operator is large and the design of a molecule that satisfies desired physical properties cannot be efficiently supported in some cases. Here, Patent Document 1 does not describe such content.

It is an object of the present disclosure to provide a design support device, a design support method, a program, and a design support system for efficiently supporting the design of a molecule that satisfies desired physical properties.

### Means for Solving the Problem

The present disclosure includes the following configurations.
[1] A design support device for supporting design of a molecule that satisfies a desired physical property, the design support device including:
   an input reception unit configured to receive an input of information on a desired physical property;
   a search unit configured to search for information on a molecule that satisfies the desired physical property, using a database storing information on molecules;
   a candidate molecule generation unit configured to generate information on a plurality of candidate molecules from a base molecule when there is no information on the molecule that satisfies the desired physical property in the database;
   a prediction unit configured to predict information on the physical property for the information on the candidate molecules; and
   a presentation unit configured to select and present the candidate molecule that satisfies the desired physical property, based on the predicted information on the physical property.
[2] The design support device as described in [1], wherein when there is no information on the molecule that satisfies the desired physical property in the database, the candidate molecule generation unit selects information on the molecule approximating the desired physical property as the base molecule, and generates the information on the plurality of candidate molecules by adjusting the selected base molecule.
[3] The design support device as described in [1] or [2], wherein the candidate molecule generation unit generates the information on the plurality of candidate molecules by adjusting a structure of the selected base molecule.
[4] The design support device as described in any one of [1] to [3], wherein the presentation unit further presents a synthetic difficulty level of the candidate molecule that satisfies the desired physical property.
[5] The design support device as described in any one of [1] to [4], further comprising a reverse synthesis analysis unit configured to perform a reverse synthesis analysis of the candidate molecule that satisfies the desired physical property,
   wherein the presentation unit presents a synthesis route of the candidate molecule subjected to the reverse synthesis analysis.
[6] The design support device as described in any one of [1] to [5], wherein the information on the physical property includes a peak position of an absorption spectrum, a half-width of the absorption spectrum, a peak position of an emission spectrum, and a half-width of the emission spectrum.
[7] The design support device as described in [6], wherein the information on the physical property includes at least one of a quantum yield, a fluorescence lifetime, or a molar absorption coefficient.
[8] A design support method performed by a computer for supporting design of a molecule that satisfies a desired physical property, the design support method including:
   an input reception step of receiving an input of information on a desired physical property;
   a search step of searching for information on a molecule that satisfies the desired physical property, using a database storing information on molecules;
   a candidate molecule generation step of generating information on a plurality of candidate molecules from a base molecule when there is no information on the molecule that satisfies the desired physical property in the database;
   a prediction step of predicting information on the physical property for the information on the candidate molecules; and
   a presentation step of selecting and presenting the candidate molecule that satisfies the desired physical property, based on the predicted information on the physical property.
[9] A program for causing a computer for supporting design of a molecule that satisfies a desired physical property to perform:
   an input reception procedure of receiving an input of information on a desired physical property;
   a search procedure of searching for information on a molecule that satisfies the desired physical property, using a database storing information on molecules;
   a candidate molecule generation procedure of generating information on a plurality of candidate molecules from a base molecule when there is no information on the molecule that satisfies the desired physical property in the database;
   a prediction procedure of predicting information on the physical property for the information on the candidate molecules; and
   a presentation procedure of selecting and presenting the candidate molecule that satisfies the desired physical property, based on the predicted information on the physical property.
[10] A design support system including a plurality of computers for supporting design of a molecule that satisfies a desired physical property, the design support system including:
   an input reception unit configured to receive an input of information on a desired physical property;
   a search unit configured to search for information on a molecule that satisfies the desired physical property, using a database storing information on molecules;
   a candidate molecule generation unit configured to generate information on a plurality of candidate molecules from a base molecule when there is no information on the molecule that satisfies the desired physical property in the database;
   a prediction unit configured to predict information on the physical property for the information on the candidate molecules; and
   a presentation unit configured to select and present the candidate molecule that satisfies the desired physical property, based on the predicted information on the physical property.

### Effect of the invention

According to the present disclosure, a design support device, a design support method, a program, and a design support system for efficiently supporting the design of a molecule that satisfies desired physical properties can be provided.

### BRIEF DESCRIPTION OF THE DRAWINGS

[FIG. 1] FIG. 1 is a configuration diagram of an example of a design support system according to the present embodiment.
[FIG. 2] FIG. 2 is a hardware configuration diagram of an example of a computer according to the present embodiment.
[FIG. 3] FIG. 3 is a functional configuration diagram of an example of a design support system according to the present embodiment.
[FIG. 4] FIG. 4 is a flowchart illustrating an example of a process of the design support system according to the present embodiment.
[FIG. 5] FIG. 5 is an image diagram of an example of a screen for receiving input of physical property information in a text format.
[FIG. 6] FIG. 6 is an image diagram of an example of a screen for receiving input of physical property information by handwriting.
[FIG. 7] FIG. 7 is a diagram for explaining an example of a database search and selection of a base molecule in step S12.
[FIG. 8] FIG. 8 is a diagram for explaining an example of a process of generating a plurality of candidate molecules from the base molecule.
[FIG. 9] FIG. 9 is a diagram for explaining an example of a molecule presented to an operator.
[FIG. 10] FIG. 10 is a diagram for explaining an example of a synthetic difficulty level of a candidate molecule.
[FIG. 11] FIG. 11 is a diagram for explaining an example of a synthetic route determined by reverse synthesis analysis of the candidate molecule.
[FIG. 12] FIG. 12 is a screen image of an example of a user terminal on which information on the candidate molecule that satisfies desired physical properties is displayed.
[FIG. 13] FIG. 13 is a screen image of an example of a user terminal on which information on the candidate molecule that satisfies desired physical properties is displayed.

### DESCRIPTION OF THE EMBODIMENTS

Next, embodiments of the present invention will be described in detail. The present invention is not limited to the following embodiments.

### [First Embodiment]

### <System Configuration>

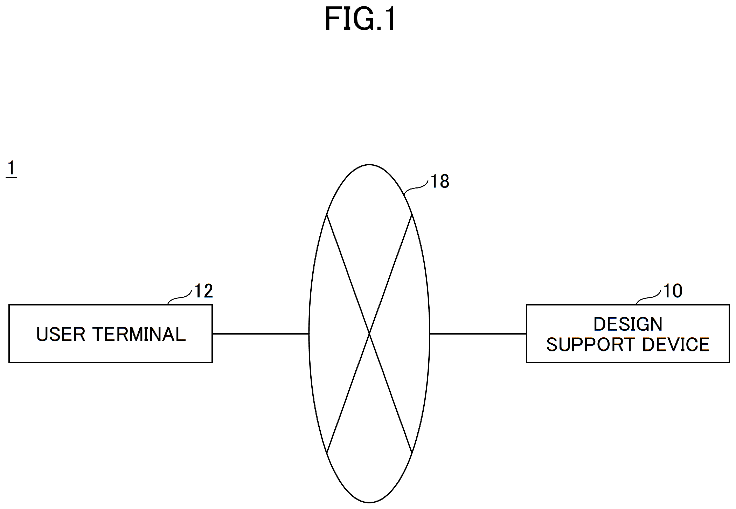

FIG. 1 is a block diagram of an example of a design support system according to the present embodiment. A design support system 1 of FIG. 1 includes a design support device 10 and a user terminal 12. The design support device 10 and the user terminal 12 are connected to each other via a communication network 18, such as a local area network (LAN) or the Internet, so that data communication can be performed.

The user terminal 12 is an information processing terminal operated by an operator, such as a PC, a tablet terminal, or a smartphone. The user terminal 12 displays a screen for receiving input of information from the operator on a display device and receives input of the information from the operator. Additionally, the user terminal 12 transmits the information received from the operator as input to the design support device 10 to perform a process of supporting the design of a molecule that satisfies desired physical properties. For example, the user terminal 12 transmits information on physical properties of a fluorescent molecule (the peak position of the absorption spectrum, the half width of the absorption spectrum, the peak position of the emission spectrum, the half width of the emission spectrum, and the like) received from the operator as input to the design support device 10, and causes the design support device 10 to perform a process of supporting the design of a molecule that satisfies the physical properties of the fluorescent molecule.

The user terminal 12 receives information on an execution result of the process of the design support device 10, displays the information on the display device, and allows the operator to confirm the information. For example, the user terminal 12 receives information on a molecule that satisfies desired physical properties received from the operator as input, displays the information on the display device, and allows the operator to confirm the information.

The design support device 10 is an information processing device such as a PC that supports the design of a molecule that satisfies desired physical properties as described later. The design support device 10 receives input of information on desired physical properties from the operator, and searches for information on a molecule that satisfies the desired physical properties, using a database. When there is no information on the molecule that satisfies the desired physical properties in the database, the design support device 10 predicts information on the physical properties of a plurality of candidate molecules generated from a base molecule, selects candidate molecules that satisfy the desired physical properties based on the predicted information on the physical properties, and presents the candidate molecules to the operator, thereby supporting the design of the molecule that satisfies the desired physical properties.

The design support device 10 utilizes a database that stores information on molecules. The design support device 10 may utilize a database stored in the device itself, or may utilize a database such as a database server connected via the communication network 18.

Additionally, the design support device 10 utilizes a mathematical model such as a machine learning model (a learned machine learning model) obtained by performing machine learning on a correspondence relationship between information on molecules and physical properties of molecules. The machine learning method uses a learning method, such as linear regression, generalized linear models, partial least squares, kernel ridge regression, Gaussian processes, k-nearest neighbor, decision tree, random forest, AdaBoost, Bagging, gradient boosting, support vector machines, or neural networks.

When predicting the information on the physical properties of the candidate molecule by the learned machine learning model, information on the molecule such as structural features of the candidate molecule is input into the learned machine learning model, and the information on the physical properties of the candidate molecule is output. The structural features of the candidate molecule can be represented by, for example, the Simplified Molecular Input Line Entry System (hereinafter referred to as SMILES) notation. The physical properties of the candidate molecule can be predicted by inputting the structural features of the candidate molecule represented by the SMILES notation into the learned machine learning model.

The design support device 10 receives information input by the operator to the user terminal 12, and performs a process of supporting the design of a molecule that satisfies desired physical properties. The design support device 10 transmits the result information of the process to the user terminal 12, and causes the user terminal 12 to display the result information of the process.

The design support system 1 of FIG. 1 may be realized by the design support device 10 having a Web server function and the user terminal 12 executing a Web application by a Web browser function. The design support system 1 of FIG. 1 may be realized by an application installed in the user terminal 12 cooperating with a program installed in the design support device 10 to perform the process.

Here, the design support system 1 of FIG. 1 is an example, and it is needless to say that there are various system configuration examples according to applications and purposes. For example, the design support device 10 may be realized by a plurality of computers or may be realized as a cloud computing service. Additionally, the design support system 1 of FIG. 1 may be realized by a stand-alone computer.

### <Hardware Configuration>

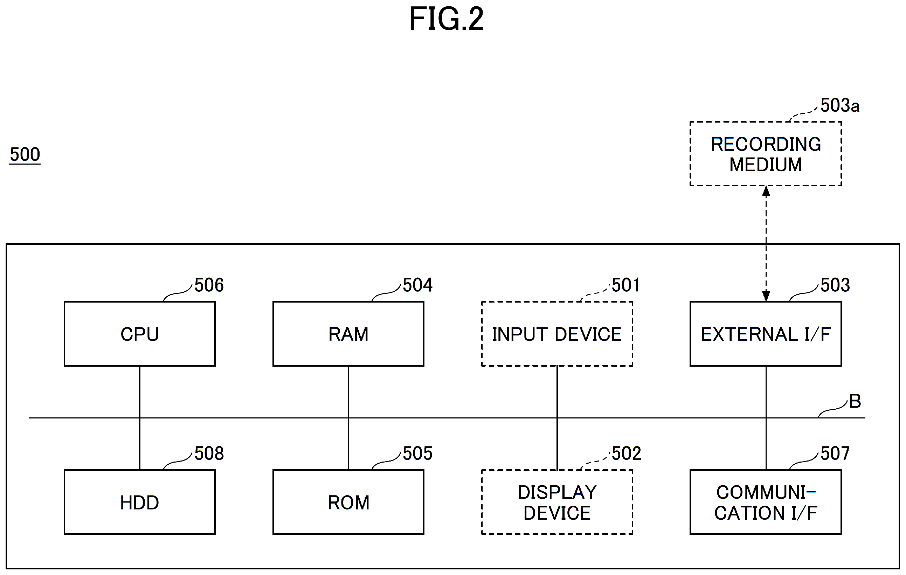

The design support device 10 and the user terminal 12 of FIG. 1 are realized by a computer 500 having a hardware configuration illustrated in FIG. 2, for example.

FIG. 2 is a hardware configuration diagram of an example of a computer according to the present embodiment. The computer 500 illustrated in FIG. 2 includes an input device 501, a display device 502, an external I/F 503, a RAM 504, a ROM 505, a CPU 506, a communication I/F 507, an HDD 508, and the like, which are mutually connected by a bus B. Here, the input device 501 and the display device 502 may be configured to be used by connecting them.

The input device 501 includes a touch panel, operation keys and buttons, a keyboard, a mouse, and the like, used by an operator to input various signals. The display device 502 includes a display, such as a liquid crystal or an organic EL for displaying a screen, a speaker for outputting sound data, such as voice and sound, and the like. The communication I/F 507 is an interface for the computer 500 to perform data communication.

Additionally, the HDD 508 is an example of a nonvolatile storage device storing programs and data. The stored programs and data include an OS, which is basic software for controlling the entire computer 500, applications for providing various functions on the OS, and the like. Here, instead of the HDD 508, the computer 500 may use a drive device (for example, a solid-state drive: SSD) using a flash memory as a storage medium.

The external I/F 503 is an interface with an external device. The external device includes a recording medium 503a and the like. With this, the computer 500 can read and/or write to the recording medium 503a via the external I/F 503. The recording medium 503a includes a flexible disk, a CD, a DVD, an SD memory card, a USB memory, and the like.

The ROM 505 is an example of a nonvolatile semiconductor memory (a storage device) that can hold programs and data even when the power is turned off. The ROM 505 stores programs and data, such as BIOS, OS settings, and network settings that are executed when the computer 500 is started. The RAM 504 is an example of a volatile semiconductor memory (a storage device) that temporarily holds the programs and data.

The CPU 506 is an arithmetic device for realizing control and functions of the entire computer 500 by reading the programs and data from the storage device, such as the ROM 505 and the HDD 508, onto the RAM 504 and performing processing. The computer 500 according to the present embodiment can realize various functions of the design support device 10 and the user terminal 12 as described later by executing programs.

### <Functional Configuration>

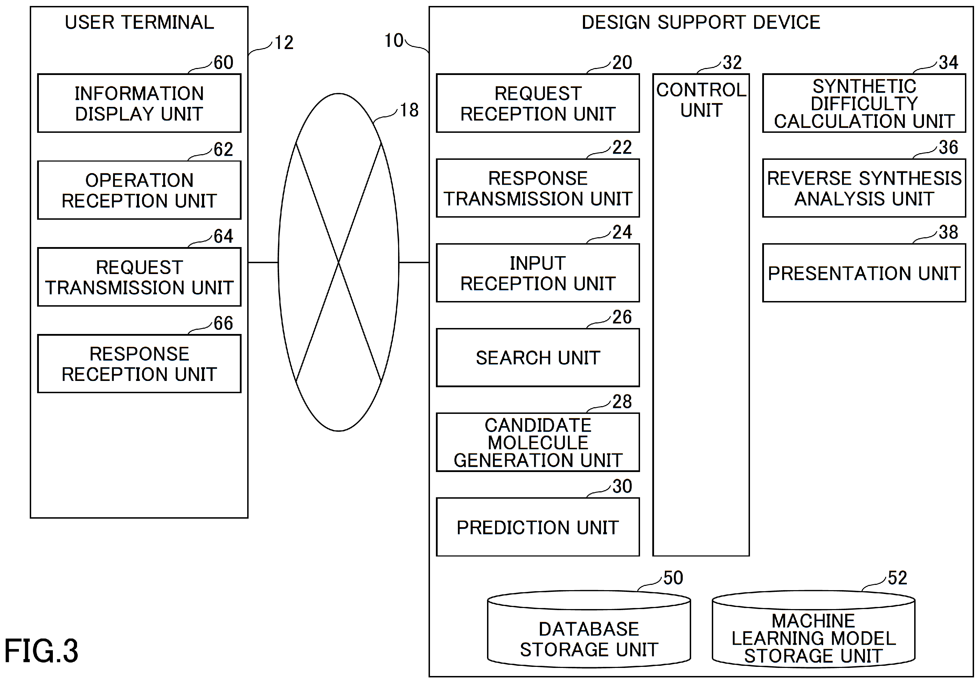

A functional configuration of the design support system 1 according to the present embodiment will be described. FIG. 3 is a functional configuration diagram of an example of the design support system according to the present embodiment. In the configuration diagram of FIG. 3, portions unnecessary for the description of the present embodiment are appropriately omitted.

The design support device 10 of the design support system 1 illustrated in FIG. 3 includes a request reception unit 20, a response transmission unit 22, an input reception unit 24, a search unit 26, a candidate molecule generation unit 28, a prediction unit 30, a control unit 32, a synthetic difficulty calculation unit 34, a reverse synthesis analysis unit 36, a presentation unit 38, a database storage unit 50, and a machine learning model storage unit 52. Additionally, the user terminal 12 includes an information display unit 60, an operation reception unit 62, a request transmission unit 64, and a response reception unit 66.

The information display unit 60 displays a screen for receiving input of information from the operator and information of the execution result of the process of the design support device 10 on the display device 502. The operation reception unit 62 receives an operation of the operator such as information input. The request transmission unit 64 transmits, to the design support device 10, a processing request corresponding to the input of the information from the operator. Additionally, the response reception unit 66 receives, from the design support device 10, a response to the processing request transmitted by the request transmission unit 64.

The request reception unit 20 receives the processing request from the user terminal 12. The response transmission unit 22 responds with the execution result of the processing corresponding to the processing request. The input reception unit 24 receives input of information on physical properties of a molecule desired by the operator in cooperation with the user terminal 12.

The search unit 26 searches for information on a molecule that satisfies the physical properties desired by the operator, using, for example, a database of information on molecules stored in the database storage unit 50. Here, the search unit 26 may use a database available via the communication network 18. When there is information on the molecule that satisfies the desired physical properties in the database, the design support device 10 finishes searching for the molecule that satisfies the desired physical properties.

When there is no information on the molecule that satisfies the desired physical properties in the database, the design support device 10 designs a molecule that satisfies the desired physical properties as follows. The candidate molecule generation unit 28 generates information on a plurality of candidate molecules from a base molecule selected as described later. The candidate molecule generation unit 28 may generate information on the plurality of candidate molecules by adjusting the structure of the selected base molecules.

The prediction unit 30 predicts information on physical properties of the plurality of candidate molecules by using, for example, a learned machine learning model stored in the machine learning model storage unit 52. Here, the prediction unit 30 may use a learned machine learning model (AI model) such as chemprop to predict the physical properties of the molecules. The prediction unit 30 may predict information on the physical properties of the plurality of candidate molecules generated by the candidate molecule generation unit 28 by using functions available via the communication network 18.

The synthetic difficulty calculation unit 34 calculates synthetic difficulty levels of the plurality of candidate molecules generated by the candidate molecule generation unit 28. The synthetic difficulty level is an index for evaluating the ease of synthesis of a molecule. The synthetic difficulty level may be calculated as a score based on, for example, the complexity of the structure of the molecule.

The reverse synthesis analysis unit 36 performs reverse synthesis analysis of the plurality of candidate molecules generated by the candidate molecule generation unit 28. The reverse synthesis analysis is a method of determining an efficient synthetic route for obtaining a target molecule. The reverse synthesis analysis is performed by rationally dividing the target molecule into precursors with simple structures. Here, the reverse synthesis analysis unit 36 may use a trained machine learning model (AI model) such as aizynthfinder, which has a function of recursively decomposing a molecule into purchasable precursors.

The presentation unit 38 selects a candidate molecule that satisfies the physical properties desired by the operator based on the information of the physical properties of the candidate molecule predicted by the prediction unit 30, and presents the selected candidate molecule to the operator by displaying the selected candidate molecule on the user terminal 12. The presentation unit 38 may present the synthetic difficulty level of the selected candidate molecule to the operator by displaying the synthetic difficulty level on the user terminal 12. Additionally, the presentation unit 38 may present the synthesis route of the selected candidate molecule to the operator by displaying the synthesis route on the user terminal 12.

The control unit 32 controls the request reception unit 20, the response transmission unit 22, the input reception unit 24, the search unit 26, the candidate molecule generation unit 28, the prediction unit 30, the control unit 32, the synthetic difficulty calculation unit 34, the reverse synthesis analysis unit 36, and the presentation unit 38 illustrated in FIG. 3.

Here, the configuration of the design support system 1 illustrated in FIG. 3 is an example. The configuration of the design support system 1 according to the present embodiment can be realized by various configurations. For example, the database storage unit 50 and the machine learning model storage unit 52 may be included in a storage device, a computer, a cloud storage, or the like that can perform data communication with the design support device 10 via the communication network 18.

### <Process>

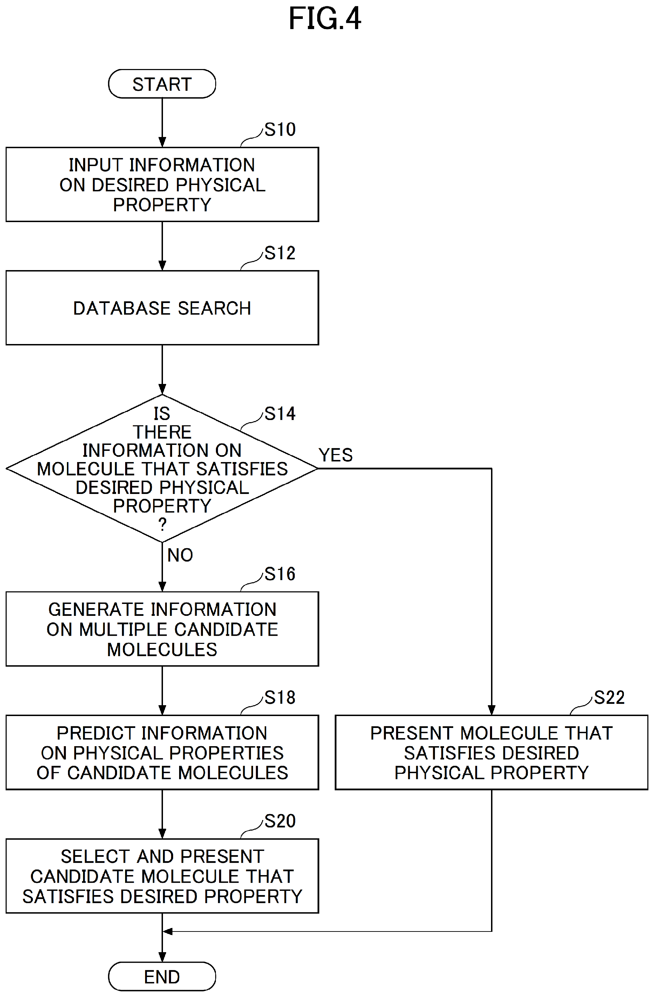

The design support system 1 according to the present embodiment supports the design of the molecule that satisfies the physical properties desired by the operator, for example, by the procedure illustrated in FIG. 4. FIG. 4 is a flowchart illustrating an example of a process of the design support system according to the present embodiment. Here, an example of supporting the design of a fluorescent molecule will be described below. Additionally, an example, in which the physical properties desired by the operator are a peak position of an absorption spectrum, a half-width of an absorption spectrum, a peak position of an emission spectrum, and a half-width of an emission spectrum, will be described below. The physical properties desired by the operator may include at least one of a fluorescence lifetime, a quantum yield, or a molar absorption coefficient.

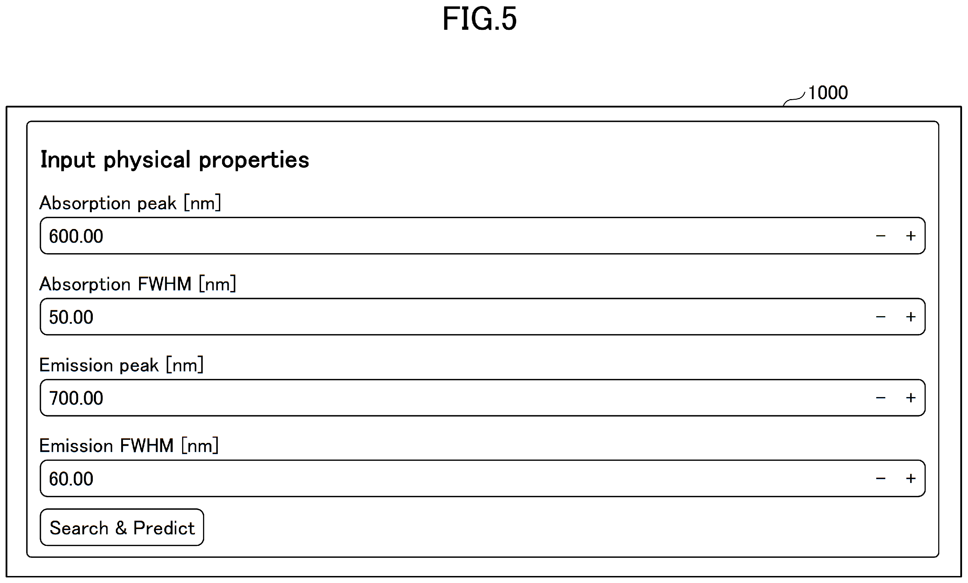

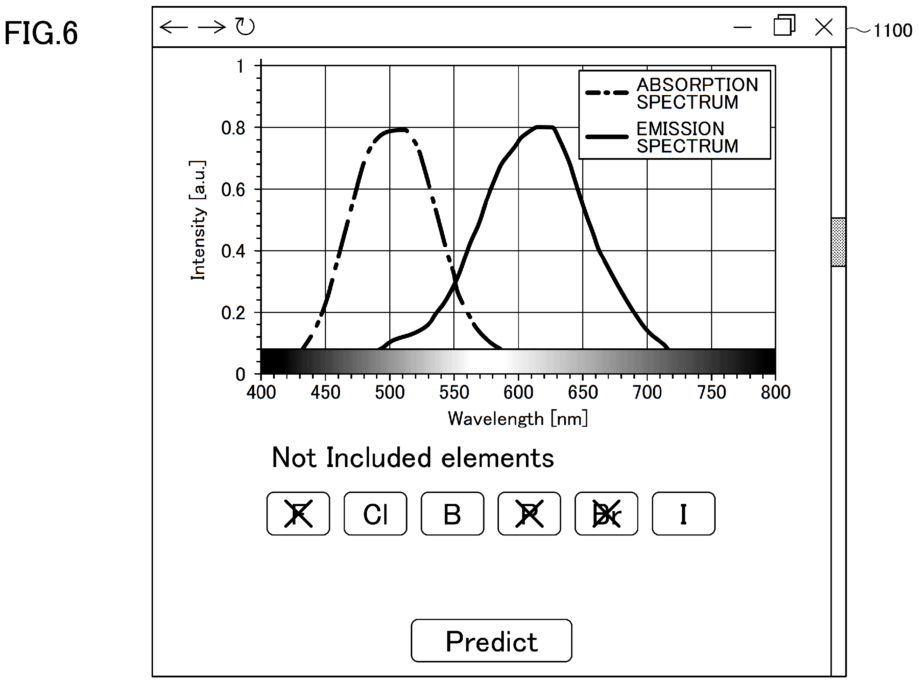

In step S10, the input reception unit 24 of the design support device 10 causes the user terminal 12 to display a screen 1000 or 1100 illustrated in FIG. 5 or FIG. 6, for example. FIG. 5 is an image diagram of an example of a screen for receiving input of information on the physical properties in a text format. FIG. 6 is an image view of an example of a screen for receiving input of information on the physical properties by handwriting.

In the screen 1000 of FIG. 5, a column for receiving, in a text format, input of the peak position of the absorption spectrum, the half-width of the absorption spectrum, the peak position of the emission spectrum, and the half-width of the emission spectrum, which are desired physical properties, is provided.

In the screen 1100 of FIG. 6, a column for receiving, by handwriting, input of the peak position of the absorption spectrum, the half-width of the absorption spectrum, the peak position of the emission spectrum, and the half-width of the emission spectrum, which are desired physical properties, is provided. Here, in the screen 1100 of FIG. 6, a column for selecting an atom not to be included is provided as an example of a constraint condition of a molecule to be designed. The input reception unit 24 receives input of the information on the physical properties desired by the operator from the screen 1000 of FIG. 5 or the screen 1100 of FIG. 6. The input reception unit 24 may draw and display the absorption spectrum and the emission spectrum from the peak position of the absorption spectrum, the half-width of the absorption spectrum, the peak position of the emission spectrum, and the half-width of the emission spectrum that are input.

In step S12, the search unit 26 searches for information on a molecule that satisfies the desired physical properties input in step S10, using a database storing information on fluorescent molecules. Here, the search target may be designated only to the peak position of the absorption spectrum. The search unit 26 may perform search weighted by the peak position of the absorption spectrum.

When performing the search weighted by the peak position of the absorption spectrum, the database storing the information on the fluorescent molecules is searched to search for information on a fluorescent molecule that satisfies the desired peak position of the absorption spectrum. When there is information on a fluorescent molecule that satisfies the desired peak position of the absorption spectrum in the database, the design support device 10 performs the process of step S22. In step S22, the presentation unit 38 presents the information on the fluorescent molecule that satisfies the peak position of the desired absorption spectrum retrieved from the database to the operator as the information on the fluorescent molecule that satisfies the desired physical property.

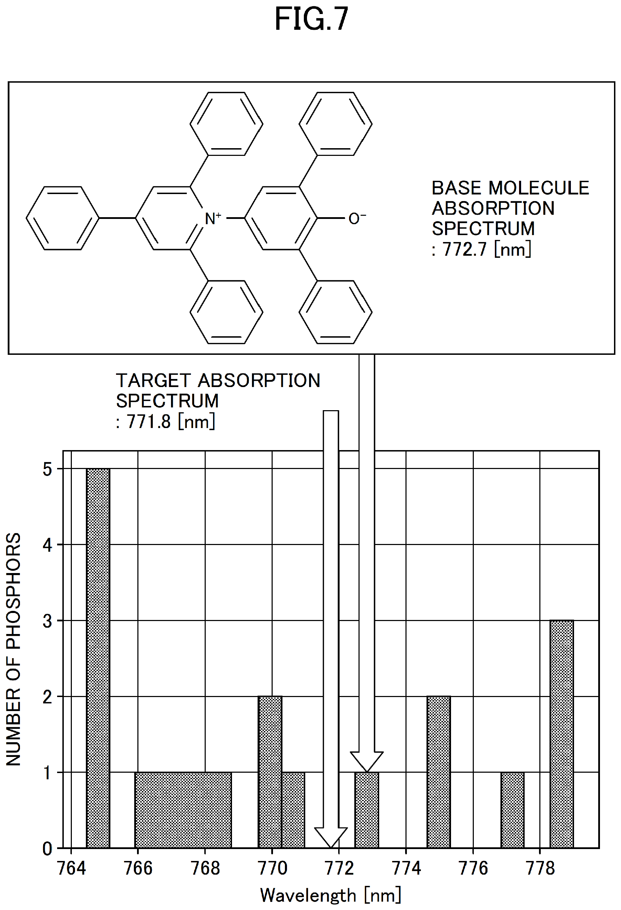

When there is no information on the fluorescent molecule that satisfies the desired peak position of the absorption spectrum in the database, the design support device 10 selects a base molecule as illustrated in FIG. 7, for example, and proceeds to the process of step S16.

FIG. 7 is a diagram for explaining an example of the database search and the selection of the base molecule in step S12. In FIG. 7, the desired peak position of the absorption spectrum is described as the target absorption spectrum. The vertical axis of the absorption histogram in FIG. 7 indicates the number of fluorescent molecules for each absorption spectrum. The horizontal axis of the absorption histogram in FIG. 7 indicates the peak wavelength of the absorption spectrum of the fluorescent molecule. The absorption histogram in FIG. 7 indicates an example in which there is no information on the fluorescent molecule that satisfies the target absorption spectrum: 771.8 [nm].

Even if the target absorption spectrum of 771.8 [nm] is not satisfied, the information on the fluorescent molecule approximating the target absorption spectrum suggests a high possibility that a fluorescent molecule that satisfies the target absorption spectrum can be obtained by adjustment. The adjustment is, for example, a change in the structure of the molecule. FIG. 7 illustrates an example in which the information on the fluorescent molecule whose absorption spectrum is 772.7 [nm] approximating the target absorption spectrum of 771.8 [nm] is selected as the base molecule. A plurality of base molecules may be selected.

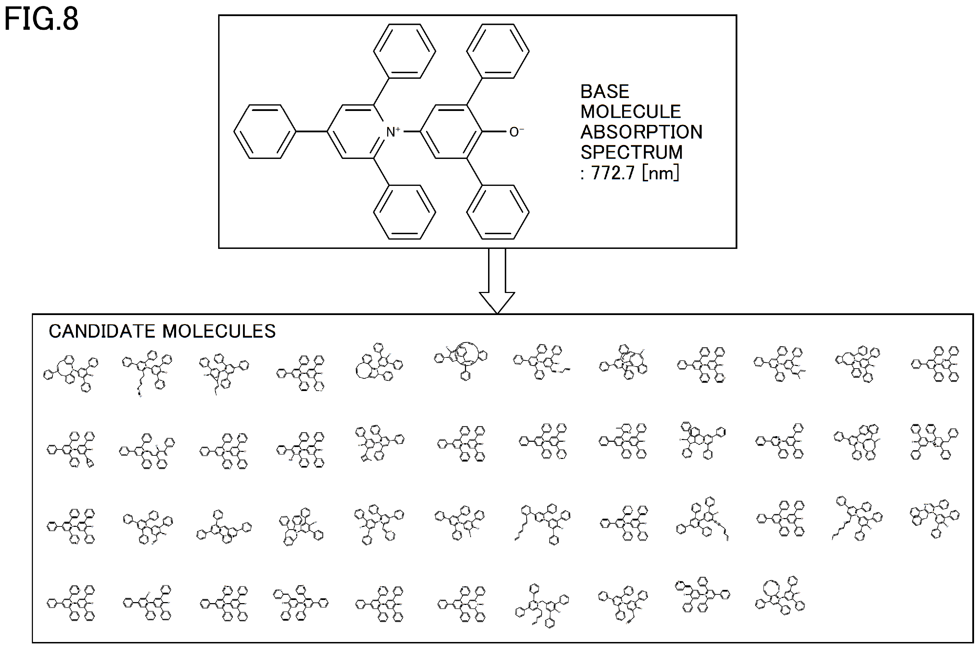

In step S16, the candidate molecule generation unit 28 generates information on a plurality of candidate molecules from the base molecules, for example, as illustrated in FIG. 8. FIG. 8 is a diagram for explaining an example of a process of generating the plurality of candidate molecules from the base molecule. The candidate molecule generation unit 28 searches for the plurality of candidate molecules generated from one or more base molecules and one or more solvents frequently used in the database storing the information on the fluorescent molecule.

In step S18, the prediction unit 30 predicts the peak position of the absorption spectrum, the half-width of the absorption spectrum, the peak position of the emission spectrum, and the half-width of the emission spectrum of each of the plurality of candidate molecules for each of the combinations with the solvents.

In step S20, the presentation unit 38 selects a candidate molecule that satisfies the peak position of the absorption spectrum, the half-width of the absorption spectrum, the peak position of the emission spectrum, and the half-width of the emission spectrum that are desired by the operator, based on the information on the peak position of the absorption spectrum, the half-width of the absorption spectrum, the peak position of the emission spectrum, and the half-width of the emission spectrum of the candidate molecule that are predicted, and displays the selected candidate molecule on the user terminal 12 to present the selected candidate molecule to the operator.

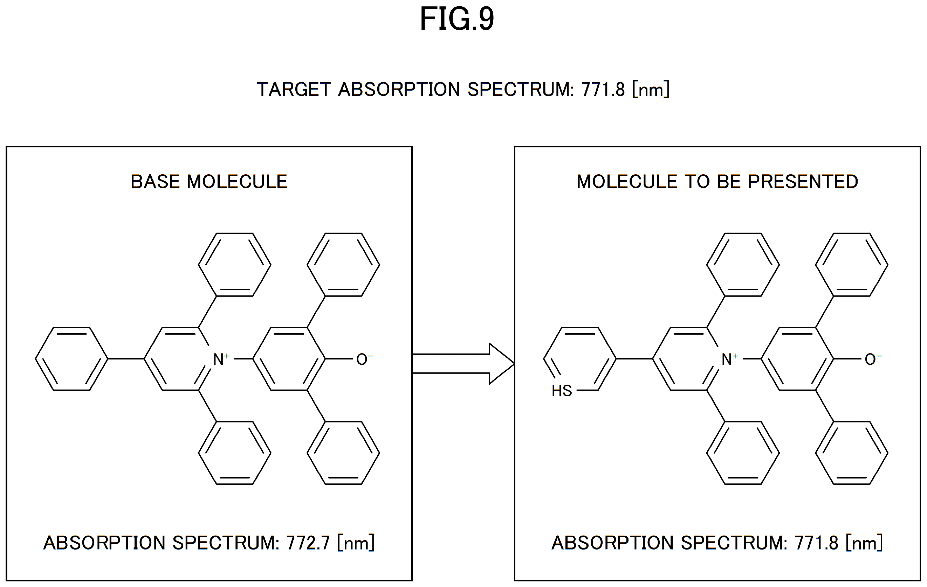

FIG. 9 is a diagram for explaining an example of a molecule presented to the operator. As illustrated in FIG. 9, the design support device 10 according to the present embodiment selects, as the base molecule, the information on the fluorescent molecule having an absorption spectrum of 772.7 [nm] that approximates the target absorption spectrum of 771.8 [nm] and selects, from the plurality of candidate molecules that approximate the base molecule, a candidate molecule that satisfies the peak position of the absorption spectrum, the half-width of the absorption spectrum, the peak position of the emission spectrum, and the half-width of the emission spectrum that are desired by the operator, thereby displaying the selected candidate molecule (the predicted value of the absorption spectrum of 771.8 [nm]) on the user terminal 12 to present the selected candidate molecule to the operator.

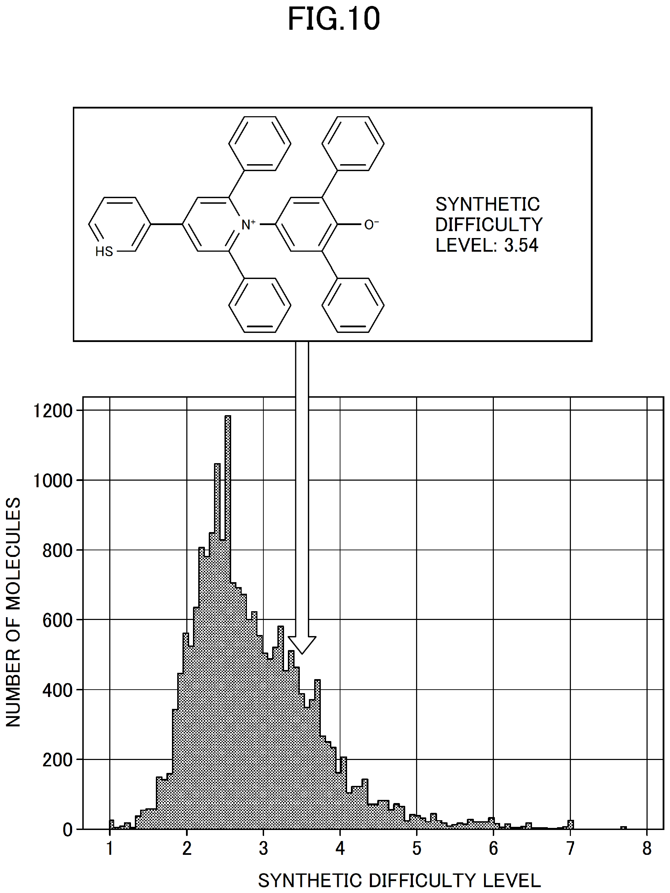

Here, in step S20, the presentation unit 38 may display the synthetic difficulty levels of the plurality of candidate molecules calculated by the synthetic difficulty calculation unit 34 together with the selected candidate molecule on the user terminal 12 to present them to the operator. FIG. 10 is a diagram for explaining an example of the synthetic difficulty levels of the candidate molecules. The vertical axis of FIG. 10 is the number of molecules. The horizontal axis of FIG. 10 is the synthetic difficulty levels. FIG. 10 indicates a frequency distribution of the synthetic difficulty levels of all molecules stored in the database. In FIG. 10, the synthetic difficulty level of the candidate molecule is displayed in comparison. For the synthetic difficulty levels of the candidate molecules, an existing index capable of evaluating the ease of synthesis of molecules is used.

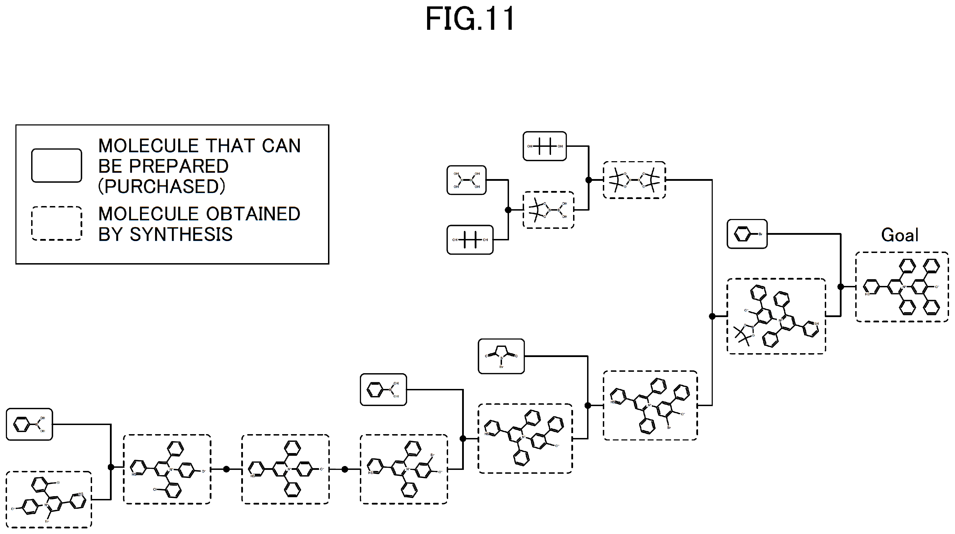

Additionally, in step S20, the presentation unit 38 may display a synthesis route of the candidate molecule subjected to the reverse synthesis analysis by the reverse synthesis analysis unit 36 together with the selected candidate molecule on the user terminal 12 to present them to the operator. FIG. 11 is a diagram for explaining an example of the synthesis route determined by reverse synthesis analysis of the candidate molecule. The synthesis route illustrated in FIG. 11 is divided into a molecule for which a candidate molecule can be prepared (purchased) and a molecule obtained by synthesis.

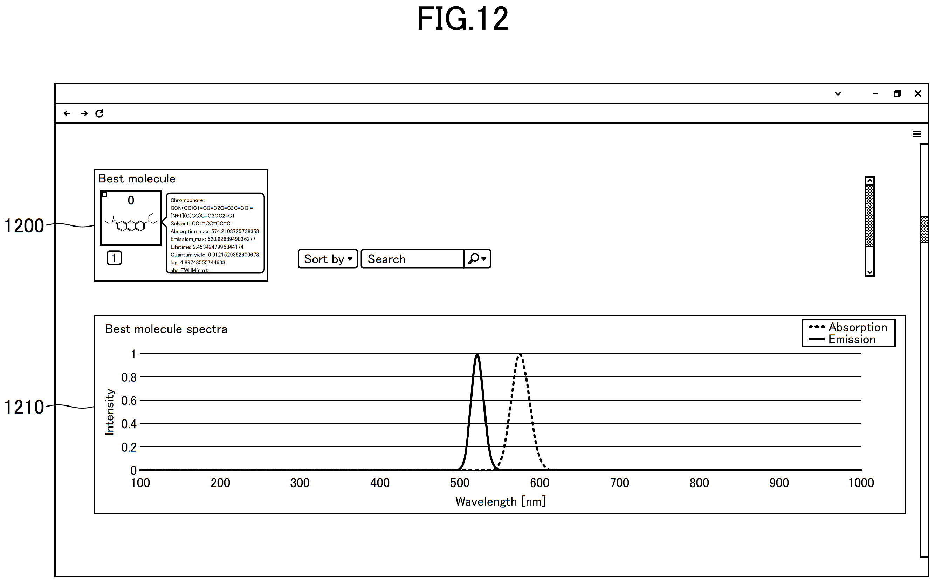

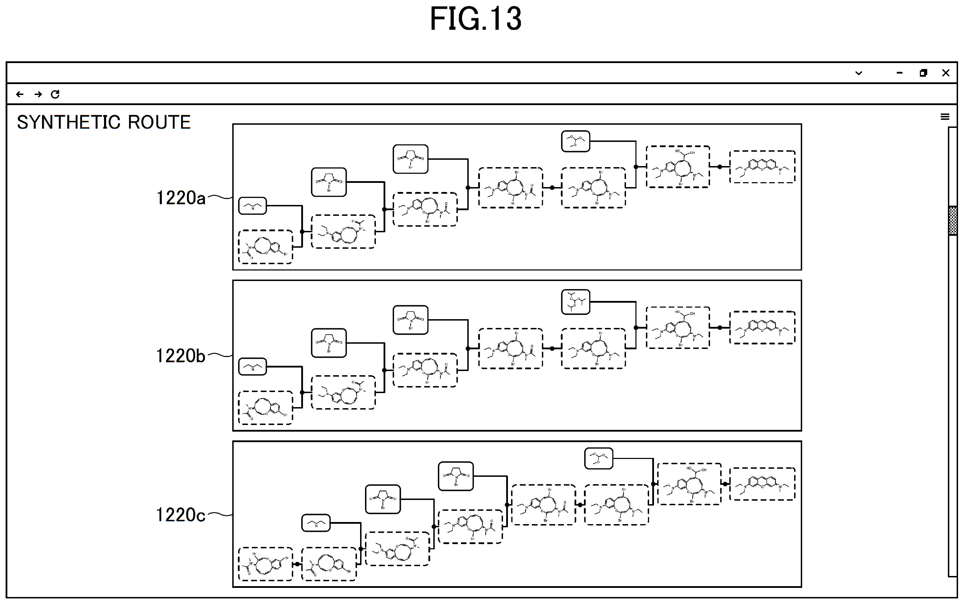

FIGS. 12 and 13 are screen images of an example of the user terminal on which the information on the candidate molecule that satisfies the desired physical properties is displayed. FIGS. 12 and 13 are examples of one Web page, and the display of FIG. 13 is obtained by scrolling the display of FIG. 12.

In a column 1200 of FIG. 12, information on the candidate molecule that satisfies the peak position of the absorption spectrum, the half-width of the absorption spectrum, the peak position of the emission spectrum, and the half-width of the emission spectrum that are desired by the operator is displayed. In a column 1210, the absorption spectrum and the emission spectrum of the candidate molecule are displayed. In columns 1220a to 1220c of FIG. 13, synthetic routes of the candidate molecule are displayed.

In addition to the information illustrated in FIGS. 12 and 13, the synthetic difficulty level of the candidate molecule, a molecular structural formula, a structural feature of the candidate molecule expressed by the SMILES notation, a fluorescence property, and a chromaticity diagram may be displayed on the screen of the user terminal 12.

### <Summary>

For example, when a molecule that satisfies physical properties desired by the operator is searched for, it is necessary to manually select a candidate molecule from among a large number of molecules and predict whether the selected candidate molecule satisfies the desired physical properties, using a machine learning model or the like. Thus, the design of a molecule that satisfies desired physical properties requires the selection of a candidate molecule from among an enormous design space, which is not efficient for the operator.

Therefore, the design support system 1 according to the present embodiment searches for information on a molecule that satisfies desired physical properties by searching a database, terminates the search when there is information on a molecule that satisfies the desired physical properties in the database, and generates a plurality of candidate molecules from a base molecule and presents the information on a molecule that satisfies the desired properties from among the candidate molecules to the operator when there is no information on a molecule that satisfies the desired physical properties in the database. The presentation to the operator may include at least one of the synthetic difficulty level or the synthetic route of the candidate molecule.

According to the design support system 1 of the present embodiment, the process from searching the database to presenting the information on the candidate molecule is seamlessly performed, and thus the design of a molecule that satisfies the desired physical properties can be efficiently supported by the operator.

For example, according to the design support system 1 of the present embodiment, the amount of work to be performed by the operator can be reduced relative to conventional approaches. Additionally, according to the design support system 1 of the present embodiment, it is easy to find a new molecule that satisfies the physical properties desired by the operator. Additionally, according to the design support system 1 of the present embodiment, the synthesis route of the candidate molecule can be presented to the operator, and the candidate molecule can be efficiently synthesized.

### [Other Embodiment]

The fluorescent molecule designed by the design support device 10 according to the present embodiment may be generated by supplying information on the synthesis route of the candidate molecule and information on the solvent to a manufacturing device configured to generate a fluorescent molecule or the like by synthesizing a plurality of raw materials.

As described above, according to the design support system 1 of the present embodiment, a design support device, a design support method, a program, and a design support system for efficiently supporting the design of a molecule that satisfies the desired physical properties can be provided.

Although the present embodiment has been described above, it will be understood that various changes in the form and details can be made without departing from the spirit and scope of the claims. For example, in the process of the flowchart of FIG. 4, when there is no information on the fluorescent molecule that satisfies the desired peak position of the absorption spectrum in the database, the information on the candidate molecule is generated. However, even when there is information on the fluorescent molecule that satisfies the desired peak position of the absorption spectrum in the database, the processes of steps S16 to S20 may be performed. Additionally, the processes of steps S12, S 14, and S22 of FIG. 4 may be omitted. If the processes of steps S12, S14, and S22 of FIG. 4 are omitted, the design support system 1 according to the present embodiment can generate information on a plurality of candidate molecules and present the information on the candidate molecule that satisfies the desired peak position of the absorption spectrum to the operator, regardless of whether there is information on the fluorescent molecule that satisfies the desired peak position of the absorption spectrum in the database or not.

Although the present invention has been described based on the embodiments, the present invention is not limited to the above embodiments, and various modifications can be made within the scope of the claims. This application is based on and claims priority to Basic Application No. 2023-145863 filed on September 8, 2023 in the Japan Patent Office, the entire contents of which are incorporated herein by reference.

### Description of reference symbols

1 design support system
10 design support device
12 user terminal
18 communication network
24 input reception part
26 search unit
28 candidate molecule generation unit
30 prediction unit
32 control unit
34 synthetic difficulty calculation unit
36 reverse synthesis analysis unit
38 presentation unit
50 database storage unit
52 machine learning model storage unit

## Claims

1. A design support device for supporting design of a molecule that satisfies a desired physical property, the design support device comprising:
an input reception unit configured to receive an input of information on a desired physical property;
a search unit configured to search for information on a molecule that satisfies the desired physical property, using a database storing information on molecules;
a candidate molecule generation unit configured to generate information on a plurality of candidate molecules from a base molecule when there is no information on the molecule that satisfies the desired physical property in the database;
a prediction unit configured to predict information on the physical property for the information on the candidate molecules; and
a presentation unit configured to select and present the candidate molecule that satisfies the desired physical property, based on the predicted information on the physical property.

2. The design support device as claimed in claim 1, wherein when there is no information on the molecule that satisfies the desired physical property in the database, the candidate molecule generation unit selects information on the molecule approximating the desired physical property as the base molecule, and generates the information on the plurality of candidate molecules by adjusting the selected base molecule.

3. The design support device as claimed in claim 1 or 2, wherein the candidate molecule generation unit generates the information on the plurality of candidate molecules by adjusting a structure of the selected base molecule.

4. The design support device as claimed in any one of claims 1 to 3, wherein the presentation unit further presents a synthetic difficulty level of the candidate molecule that satisfies the desired physical property.

5. The design support device as claimed in any one of claims 1 to 4, further comprising a reverse synthesis analysis unit configured to perform a reverse synthesis analysis of the candidate molecule that satisfies the desired physical property,
wherein the presentation unit presents a synthesis route of the candidate molecule subjected to the reverse synthesis analysis.

6. The design support device as claimed in any one of claims 1 to 5, wherein the information on the physical property includes a peak position of an absorption spectrum, a half-width of the absorption spectrum, a peak position of an emission spectrum, and a half-width of the emission spectrum.

7. The design support device as claimed in claim 6, wherein the information on the physical property includes at least one of a quantum yield, a fluorescence lifetime, or a molar absorption coefficient.

8. A design support method performed by a computer for supporting design of a molecule that satisfies a desired physical property, the design support method comprising:
an input reception step of receiving an input of information on a desired physical property;
a search step of searching for information on a molecule that satisfies the desired physical property, using a database storing information on molecules;
a candidate molecule generation step of generating information on a plurality of candidate molecules from a base molecule when there is no information on the molecule that satisfies the desired physical property in the database;
a prediction step of predicting information on the physical property for the information on the candidate molecules; and
a presentation step of selecting and presenting the candidate molecule that satisfies the desired physical property, based on the predicted information on the physical property.

9. A program for causing a computer for supporting design of a molecule that satisfies a desired physical property to perform:
an input reception procedure of receiving an input of information on a desired physical property;
a search procedure of searching for information on a molecule that satisfies the desired physical property, using a database storing information on molecules;
a candidate molecule generation procedure of generating information on a plurality of candidate molecules from a base molecule when there is no information on the molecule that satisfies the desired physical property in the database;
a prediction procedure of predicting information on the physical property for the information on the candidate molecules; and
a presentation procedure of selecting and presenting the candidate molecule that satisfies the desired physical property, based on the predicted information on the physical property.

10. A design support system including a plurality of computers for supporting design of a molecule that satisfies a desired physical property, the design support system comprising:
an input reception unit configured to receive an input of information on a desired physical property;
a search unit configured to search for information on a molecule that satisfies the desired physical property, using a database storing information on molecules;
a candidate molecule generation unit configured to generate information on a plurality of candidate molecules from a base molecule when there is no information on the molecule that satisfies the desired physical property in the database;
a prediction unit configured to predict information on the physical property for the information on the candidate molecules; and
a presentation unit configured to select and present the candidate molecule that satisfies the desired physical property, based on the predicted information on the physical property.
